# EUROPEAN PATENT SPECIFICATION

(11) **EP 0 948 644 B1**
(45) Date of publication and mention of the grant of the patent: **15.01.2003**
(21) Application number: 97941979.3
(22) Date of filing: 05.09.1997
(51) Int. Cl.: C12Q 1/68

(54) **PLASMIDS CONTAINING TWO OR MORE COMPETITORS IN SEQUENCE AND THEIR APPLICATION IN COMPETITIVE-PCR TECHNIQUES**
PLASMID MIT 2 ODER MEHREREN SEQUENZKOMPETITOREN UND DEREN ANWENDUNG IN KOMPETITIVEN PCR TECHNIKEN
PLASMIDES CONTENANT DEUX OU PLUSIEURS ELEMENTS COMPETITIFS EN SEQUENCE ET LEUR APPLICATION DANS DES TECHNIQUES COMPETITIVES DE REACTION EN CHAINE DE LA POLYMERASE

(30) Priority: 05.09.1996 IT FI960208
(43) Date of publication of application: 13.10.1999
(73) Proprietor: Orlando, Claudio, 50142 Firenze (IT); Pazzagli, Mario, 50144 Firenze (IT); Sestini, Roberta, 50141 Firenze (IT); Serio, Mario, 50012 Bagno A Ripoli (IT)
(72) Inventor: Orlando, Claudio, 50142 Firenze (IT); Pazzagli, Mario, 50144 Firenze (IT); Sestini, Roberta, 50141 Firenze (IT); Serio, Mario, 50012 Bagno A Ripoli (IT)
(74) Representative: Gervasi, Gemma, Dr.
(86) International application number: EP9704814
(87) International publication number: WO98010094

(56) References cited:
- WO-A-93/13130
- WO-A-94/20640
- US-A- 5 476 774
- HAHN M ET AL: "QUANTITATIVE POLYMERASE CHAIN REACTION WITH ENZYME-LINKED IMMUNOSORBENT ASSAY DETECTION OF SELECTIVELY DIGESTED AMPLIFIED SAMPLE AND CONTROL DNA" ANALYTICAL BIOCHEMISTRY, vol. 229, no. 2, 10 August 1995, pages 236-248, XP000533292
- VENE T ET AL: "QUANTIFICATION OF HIV-1 USING MULTIPLE COMPETITORS IN A SINGLE-TUBE ASSAY" BIOTECHNIQUES, vol. 21, no. 2, August 1996, pages 248-250, 252, 254/255, XP000616031
- LUNDWALL AKE: "Characterization of the gene for prostate-specific antigen, a human glandular kallikrein" BIOCHEM. BIOPHYS. RESEARCH COMMUN., vol. 161, no. 3, - 30 June 1989 pages 1151-1159, XP002051532
- SESTINI R. ET AL.,: "gene amplification for c-erbB-2, c-myc, epidermal growth factor receptor, int-2, and N-myc measured by quantitative PCR with multiple competitor template" CLIN. CHEMISTRY, vol. 41, no. 6, - 1995 pages 826-832, XP002051540 cited in the application

### SEQUENCE LISTING

(1) GENERAL INFORMATION:
   (i) APPLICANT:
      (A) NAME: ORLANDO CLAUDIO
      (B) STREET: VIA DELLA FONDERIA 19
      (C) CITY: FIRENZE
      (D) STATE: FIRENZE
      (E) COUNTRY: ITALY
      (F) POSTAL CODE (ZIP): 50142

      (A) NAME: PAZZAGLI MARIO
      (B) STREET: VIA PIERLUIGI DA PALESTRINA 9
      (C) CITY: FIRENZE
      (D) STATE: FIRENZE
      (E) COUNTRY: ITALY
      (F) POSTAL CODE (ZIP): 50144

      (A) NAME: SESTINI ROBERTA
      (B) STREET: VIA REGINALDO GIULIANI 126
      (C) CITY: FIRENZE
      (D) STATE: FIRENZE
      (E) COUNTRY: ITALY
      (F) POSTAL CODE (ZIP): 50141

      (A) NAME: SERIO MARIO
      (B) STREET: VIA DI BARONCELLI 29
      (C) CITY: BAGNO A RIPOLI
      (D) STATE: FIRENZE
      (E) COUNTRY: ITALY
      (F) POSTAL CODE (ZIP): 50012
   (ii) TITLE OF INVENTION: PLASMIDS CONTAINING TWO OR MORE COMPETITORS IN SEQUENCE AND THEIR APPLICATION IN COMPARATIVE-PCR TECHNIQUES
   (iii) NUMBER OF SEQUENCES: 32
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi): SEQUENCE DESCRIPTION: SEQ ID NO: 1:
(2) INFORMATION FOR SEQ ID NO: 2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 2:
   TTCTAGGAGC TTCTGTGCAT CATAGTATCC 30
(2) INFORMATION FOR SEQ ID NO: 3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO: 3:
   TGCCCACTGC ATCAGGAA 18
(2) INFORMATION FOR SEQ ID NO: 4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 4:
   CCTTGAAGCA CACCATTACA 20
(2) INFORMATION FOR SEQ ID NO: 5:
   (i) SEQUENCE CHARACTERISTICS:
      (A) LENGTH: 30 base pairs
      (B) TYPE: nucleic acid
      (C) STRANDEDNESS: single
      (D) TOFOLOGY: linear
   (xi) SEQUENCE DESCRIPTION: SEQ ID NO: 5:
   AGGGGCCAAA GGAGTAATTC TCTACTCCGA 30
(2) INFORMATION FOR SEQ ID NO: 6:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 6:
   TTCTAGGAGC TTCTGTGCAT CATAGTATCC 30
(2) INFORMATION FOR SEQ ID NO: 7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 7:
   ACACAGGCCA GGTATTTCAG 20
(2) INFORMATION FOR SEQ ID NO: 8:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO: 8:
   TGCCCACTGC ATCAGGAA 18
(2) INFORMATION FOR SEQ ID NO: 5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 9:
(2) INFORMATION FOR SEQ ID NO: 10:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 10:
   AAGCCAATGC GGTTGTTACG C 21
(2) INFORMATION FOR SEQ ID NO: 11:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 11:
   ATGTCTAGGG TCTAGACATG TTCA 24
(2) INFORMATION FOR SEQ ID NO: 12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 12:
   CTTTGCCGTT GTCGCAGACG 20
(2) INFORMATION FOR SEQ ID NO: 13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
         (xi) SEQUENCE DESCRIPTION: SEQ ID NO: 13:
   TGACCACCCA GCCATCCTTC 20
   (2) INFORMATION FOR SEQ ID NO: 14:
      (i) SEQUENCE CHARACTERISTICS:
         (A) LENGTH: 21 base pairs
         (B) TYPE: nucleic acid
         (C) STRANDEDNESS: single
         (D) TOPOLOGY: linear
      (xi) SEQUENCE DESCRIPTION: SEQ ID NO: 14:
   GAGGAGGTGT TGACTTCATT C 21
   (2) INFORMATION FOR SEQ ID NO: 15:
      (i) SEQUENCE CHARACTERISTICS:
         (A) LENGTH: 28 base pairs
         (B) TYPE: nucleic acid
         (C) STRANDEDNESS: single
         (D) TOPOLOGY: linear
      (xi) SEQUENCE DESCRIPTION: SEQ ID NO: 15:
   CGCGGATCCA GGAGTACCCT GATATGAG 28
   (2) INFORMATION FOR SEQ ID NO: 16:
      (i) SEQUENCE CHARACTERISTICS:
         (A) LENGTH: 25 base pairs
         (B) TYPE: nucleic acid
         (C) STRANDEDNESS: single
         (D) TOPOLOGY: linear
      (xi) SEQUENOE DESCRIPTION: SEQ ID NO: 16:
(2) INFORMATION FOR SEQ ID NO: 17:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO: 17:
   ATGGATTATT TGCTCATGAT TTT 23
(2) INFORMATION FOR SEQ ID NO: 18:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO: 18:
   CAGTCTTTCT CCATAATGTC TTCAGC 26
(2) INFORMATION FOR SEQ ID NO: 19:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 19:
   CCTTTTGATC ACAATGACTT T 21
(2) INFORMATION FOR SEQ ID NO: 20:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEC ID NO: 20:
   TTTGATGTGG CATTGACCAT ACAG 24
(2) INFORMATION FOR SEQ ID NO: 21:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 21:
   CCTGCACTTC CAGGAGCGCA C 21
(2) INFORMATION FOR SEQ ID NO: 22:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 22:
   GCCTTGGACA CCAACTATTG C 21
(2) INFORMATION FOR SEQ ID NO: 23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 23:
   GCACTGGAAC TTACAACACC 20
(2) INFORMATION FOR SEQ ID NO: 24:
   (i) SEQUENCE CHARACTERISTICS :
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 24:
   AAGCTAACGT TGAGGGGCAT 20
(2) INFORMATION FOR SEQ ID NO: 25:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 25:
   TTGGAGGACC TGGTGGTGGC CCAC 24
(2) INFORMATION FOR SEQ ID NO: 26:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 26:
   AAAGTTGATG TCATGCTCGT CGCT 24
(2) INFORMATION FOR SEQ ID NO: 27:
   (i) SEQUENCE CHARACTERISTICS:
      (A) LENGTH; 29 base pairs
      (B) TYPE: nucleic acid
      (C) STRANDEDNESS: single
      (D) TOPOLOGY: linear
   (xi) SEQUENCE DESCRIPTION: SEQ ID NO: 27:
(2) INFORMATION FOR SEQ ID NO: 28:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO: 28:
   TGAACAATAG CCAGGTATCG ATCAATGC 28
(2) INFORMATION FOR SEQ ID NO: 29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 29:
   GGAACTCTAT GGTCATCTAC 20
(2) INFORMATION FOR SEQ ID NO: 30:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 30:
   GCTGAGCACA GTCAGACAGT 20
(2) INFORMATION FOR SEQ ID NO: 31:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 31:
   CAGTCATGAG CATCGACCGA 20
(2) INFORMATION FOR SEQ ID NO: 32:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 32:
   GCAAAGACAG ATGATGGTGA 20

## Description

The present invention refers to plasmids acting as multiple competitors for use in PCR amplification technique, that allow to quantitate simultaneously two or more genic sequences by competitive PCR technique.

### State of the art

Polymerase Chain Reaction (PCR) is a well assessed "in vitro" amplification technique which allows the exponential production of a double strand DNA fragment starting from DNA or RNA sequences. Since the quantity of the final amplified products is not only depending on the number of target molecules in the initial sample, but also on a wide range of uncontrollable and unpredictable variables, this technique is not suitable, in its conventional application, to provide quantitative results.

To overcome this problem and to obtain quantitative data, a technique known as competitive-PCR was set up. This technique is based on the simultaneous PCR amolification of the desired genomic target and of a competitor target acting as internal standard. The competitor has a molecular size similar to genomic target and share with it the same primers recognition sites, but it contains a mutation (insertion or deletion of some nucleotides) which allows an easy resolution after gel electrophoresis and staining with ethidium bromide. As competitor and target sequence share the same primers recognition sites, the final ratio of PCR products reflects their ratio in the initial sample and therefore the concentration of the target sequence can be easily deduced, since the molar concentration of the competitor in each test tube is known

In spite of the important advantages of this technique, one of its practical problems is that till now synthetic competitors allowed only single assays in the same batch, even when the sequence of primers pairs of different genes were cloned in the same plasmid vector (and therefore acting as multiple competitors) it was possible to obtain reagents allowing the recognition and measurement of a single DNA or mRNA sequence in each experiment [Clin. Chem. 41/6, 826-832: 1995].

US-A-5,476,774 describes i.e. a plasmid useful for providing an internal standard for quantitation of target nucleic acid segments, said plasmids comprising a DNA sequence comprising sequences identical to DNA sequences contained within the target nucleic acid segments, anyhow also in this case only the recognition and measurement of a single DNA or mRNA sequence in each performed test.

### Summary of the invention

Plasmid containing two or more competitors in sequence, each competitor containing the recognition sequences of one or two primers pairs and its use for the simultaneous determination and quantitation of the expression of one or more genes by competitive-PCR techniques.

### Brief description of the drawings

Figure 1 shows the construction of a plasmid containing a single primers pair for each competitor A and B.
Figure 2 describes the construction of a plasmid wherein each competitor contains two primers pairs.
Figure 3 shows a scheme of the simultaneous assay of two genes (A and B) in two samples (1 and 2) by multiplex competitive PCR.

### Detailed description of the invention

The present invention allows the quantitation of different DNA or mRNA sequences in the same tube and therefore to overcome the problem discussed above thanks to a plasmid acting as multiple competitor for use in PCR amplification containing in sequence separated from each other, two or more competitors homologous to that of the cellular target, each competitor containing the recognition sequences of one or two primers pairs.

Each competitor has the same recognition sites of the primers of the corresponding target, but the DNA fragment obtained after PCR cycling have different size (a different number of nucleotides of the two DNA species permits to identify target and competitor species after gel electrophoresis). Furthermore the cloned competitors have different size to make possible the identification of different DNA species in the same reaction tube (multiple competitive PCR).

It is obvious that the possibility of making two or more measurement of the expression of different genes (in many cases acting together in co-ordinating the physiological and pathological function of a tissue) by utilising a single plasmid containing different competitors in sequence, according to the described invention, provides a relevant improvement of the accuracy of the assay as a whole. In addition, the simultaneous measurement of more gene transcripts reduces the assay time lengtn and its cost making possible a larger number of determinations, in particular when the amount of starting biological material is limited (as in the case of needle biopsies).

Obviously the lengths of competitors can be chosen according to the need and, on the other hand, the size of target DNA and mRNA can be chosen according to a wide range of criteria; moreover each competitor can contain not only one but also two primers pairs.

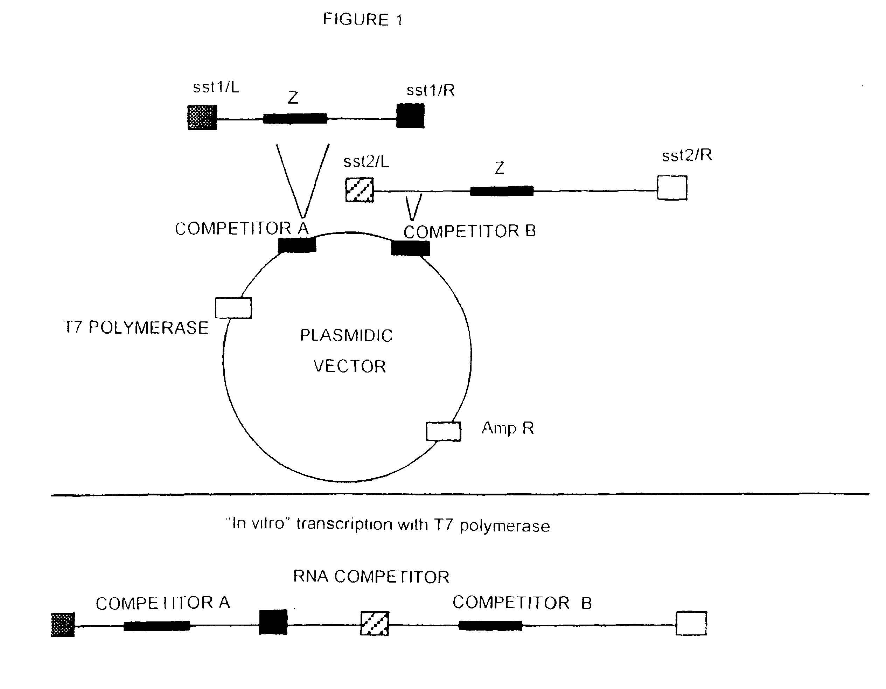

In Fig 1 it is shown the construction of a plasmid that, according to the invention contains a single primers pair for each competitor In competitors A and B the boxes representing PCR primers have the same sequence of the corresponding target but differ from its sequence for a short insertion [represented by the black-box (Z)]. The competitors are cloned in the same plasmid (cloning vector), following the dealer's instructions, down-stream of the recognition sequence of T7 polymerase, a DNA-dependent RNA polymerase which allows the transcription in vitro' of RNA competitors for the assay. The transcription 'in vitro' with T7 polymerase provides a single RNA fragment, containing the cloned competitors which can be utilized in competitive RT-PCR assays, in the presence of two or more primers pairs.

In particular Fig 1 refers to the construction of a plasmid which allows the simultaneous evaluation of gene expression of two sub-types of somatostatin receptor (sst1 and sst2 respectively, see table 1 for the description of the primers) which represent two autonomous biological entities differently distributed in normal and pathological tissues, with different clinical and physiopathological implications. The plasmid prepared according this scheme allows the simultaneous measurement of the expression of the two genes starting from a single sample and their comparison In the same reaction tube.

According to a particular embodiment of the invention, the above described competitor can be used in nested-PCR technique in which, in the first phase, the desired sequence is amplified with a traditional PCR by a pair of external primers and then an aliquot of the amplified products is used for a second PCR in the presence of two primers internal to the sequence amplified in the first cycle. This technique is necessary when very low mRNA quantities have to be revealed in blood or tissues. Some of the best known examples of this application are the recognition of HIV or HCV in blood or the method to reveal circulating cancer cells in blood stream.

Even in this case the use of a plasmid containing competitors with modified sequences, but recognized by the same primers pairs utilized in the two phases of nested-PCR, offers considerable advantages. In fact, by the addition of limited amounts of cloned competitor, an accurate and sensitive assay of low expressed mRNAs will be possible With regard to the advantages of the application of this type of competitors, it must be considered that the classical nested-PCR allows to recognize one cancer cell among 100.000 normal cells, but it can not provide a quantitative analysis as obtainable by the technique above described.

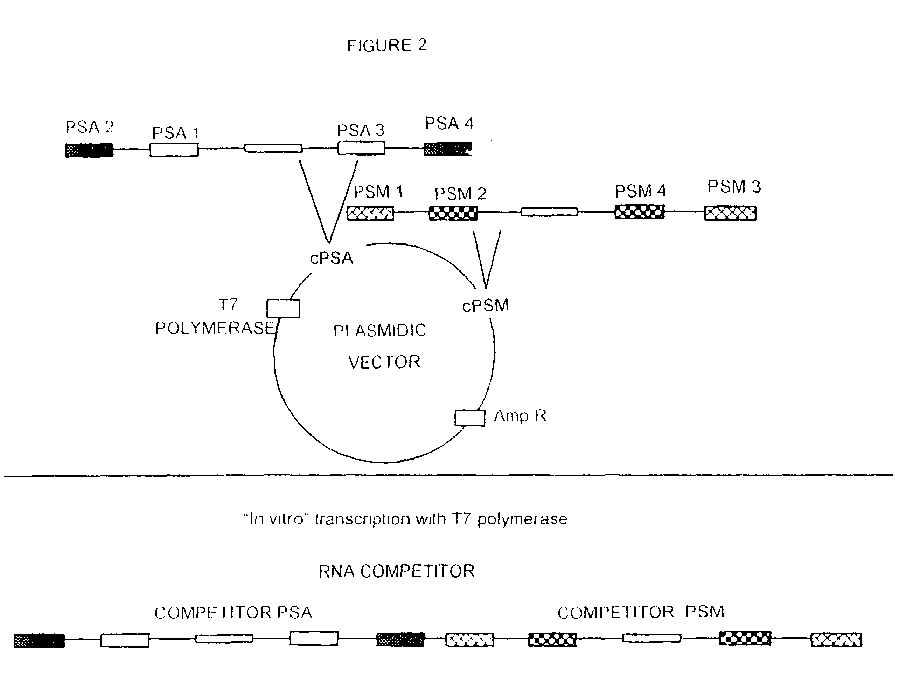

Figure 2 describes the construction of a plasmid according to the invention in which each competitor contains two primers pairs. In particular this example is referred to a plasmid capable of simultaneously measuring the expression of two prostatic antigens (PSA and PSM) that, for their organ specificity, allow to identify and measure the presence of cancer prostatic cells in venous blood stream.

The competitor structure and the resulting plasmid are reported in Fig 2 wherein PSM2, PSM3, PSA2 and PSA4 represent the following external primers, respectively and PSM1, PSM4, PSA1 and PSA3 represent respectively the following internal primers

The plasmidic vector is a cloning plasmid (pCRII produced by Invitrogen. Inc )

With a similar procedure, but utilizing primers sequences for different genes it is possible to prepare plasmids which are utilized for the simultaneous and quantitative determination of such genes A list of primers and of the corresponding genes is reported in TABLE 1, which contains also the size of amplified products.

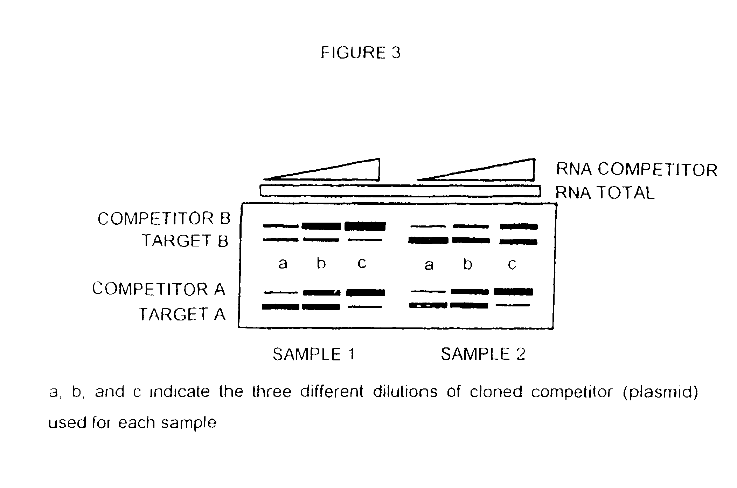

Figure 3 shows a scheme of the simultaneous assay of two genes (A and B) in two samples (1 and 2) by multiplex competitive PCR using the plasmid according to the invention, as described in Figure 1. As reported, using the described plasmid (containing two competitors with their respective primers pairs) it is possible to amplify simultaneously four sequences (the two targets and their corresponcing competitors) generating four PCR products for each tube, revealable by electrophoresis.

Since the plasmid was added at the beginning of the reaction in three different amounts (a, b and c) it is possible to perform a determination by multiplex competitive RT-PCR in the same sample, obtaining an immediate comparison between the expression levels of two different genic species, evaluating the ratio of electrophoretic bands corresponding to target and corresponding competitors

### Determination of gene expression levels

The preparation and the use of multiple competitor for the assay of the expression of gene involved in sclerosis of human tissues and organs caused by the anomalous production of extra-cellular matrix, can be applied to clinical and pharmacological studies in several pathologies caused by organ fibrosis, for example for studying nephrosclerosis). The monitoring in a needle biopsy of the renin-angiotensin II cascade and growth factors (TGF-b and PDGF, in particular), which can induce sclerosis, could be of large interest in monitoring the evolution of pathologies causing functional reduction in kidney.

Another application concerns the somatostatin receptors (sst1/sst2). It is well known tnat tne stimulation of these receptors with hypothalamic hormone analogs has artitumoral effects, but it is extremely difficult to predict which patients can obtain a positive effect from the treatment, specially because there are many different receptor subtypes each showing a different receptorial affinity for the drug. The treatment in patients with high levels of specific receptor for a somatostatin analog could provide extremely positive results in metastatic prostate carcinomas, resistant to antiandrogen therapy. The quantity of somatostatin receptor expression can be measured by competitive PCR in biological material obtained by needle biopsy.

A further application could be the quantitative evaluation of neoplastic cells in blood stream, in bronchial aspirates, in peritoneal and/or pleuric liquid of neoplastic patients.

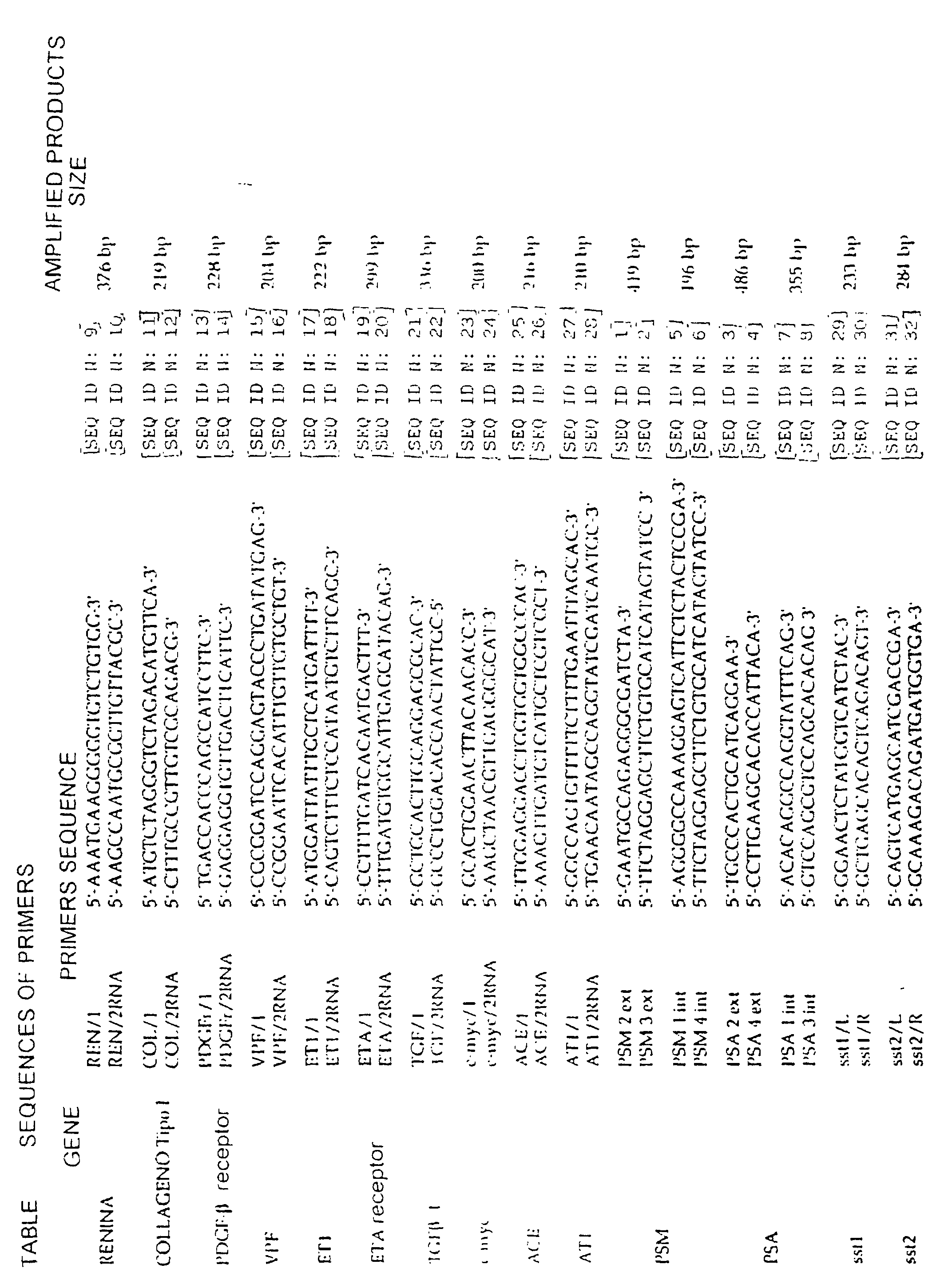

## Claims

1. Plasmid acting as multiple competitor for use in PCR amplification containing in sequence separated from each other, two or more competitors homologous to that of the cellular target, each competitor containing the recognition sequences of one or two primers pairs.

2. Plasmid according to claim 1 wherein each competitor contains the recognition sequences of one primers pair.

3. Plasmid according to claim 2 containing two competitors.

4. Plasmid according to claim 3 wherein a competitor contains the following primers pair: and the second competitor contains the following primers pair:

5. Plasmid according to Claim 1 wherein each competitor contains the recognition sequences of two primers pairs.

6. Plasmid according to Claim 5 containing two competitors.

7. Plasmid according to Claim 6 wherein a competitor contains the two following primers pairs: (external primers)
and (internal primers)'
and the second competitor contains the two following primers pairs: (external primers)
and (internal primers)

8. Use of a plasmid according to Claim 1 for the simultaneous determination and quantitation of the expression of one or more genes by competitive-PCR techniques.

9. Use of a plasmid according to Claim 4 for the simultaneous determination and quantitation of the expression of type-1 and type-2 receptors of somatostatine.

10. Use of the plasmide according to claim 7 for the simultaneous determination and quantitation of the expression of prostatic antigenes PSA and PSM.

11. Method for the simultaneous determination and quantitation of the expression of two or more genes IN COMPETITIVE-PCR TECHNIQUES by using a plasmid according to claim 1.

## Patentansprüche

1. Ein Plasmid, welches als multipler Kompetitor zur Verwendung bei der PCR-Amplifikation dient, welches in einer Reihe getrennt von einander zwei oder mehr Kompetitoren enthält, die homolog zu dem des zellularen Ziels sind, wobei jeder Kompetitor die Erkennungssequenzen von einem oder zwei Primerpaaren enthält.

2. Ein Plasmid nach Anspruch 1, in welchem jeder Kompetitor die Erkennungssequenz eines Primerpaares enthält.

3. Ein Plasmid nach Anspruch 2, welches zwei Kompetitoren enthält.

4. Ein Plasmid nach Anspruch 3, in welchem ein Kompetitor das folgende Primerpaar enthält: und der zweite Kompetitor das folgende Primerpaar enthält:

5. Ein Plasmid nach Anspruch 1, in welchem jeder Kompetitor die Erkennungssequenz von zwei Primerpaaren enthält.

6. Ein Plasmid nach Anspruch 5, welches zwei Kompetitoren enthält.

7. Ein Plasmid nach Anspruch 6, in welchem ein Kompetitor die folgenden beiden Primerpaare enthält: (externer Primer)
und (interner Primer)
und der zweite Kompetitor die folgenden beiden Primerpaare enthält: (externer Primer) und (interner Primer)

8. Verwendung eines Plasmids nach Anspruch 1 für die simultane Bestimmung und Quantifizierung der Expression von einem oder mehr Genen bei kompetitiven PCR-Techniken.

9. Verwendung eines Plasmids nach Anspruch 4 für die simultane Bestimmung und Quantifizierung der Expression von Typ-1 und Typ-2-Rezeptoren von Somatostatin.

10. Verwendung eines Plasmids nach Anspruch 7 für die simultane Bestimmung und Quantifizierung der Expression der Prostata-Antigene PSA und PSM.

11. Ein Verfahren zur simultanen Bestimmung und Quantifizierung der Expression von zwei oder mehr Genen bei kompetitiven PCR-Techniken unter Verwendung eines Plasmids nach Anspruch 1.

## Revendications

1. Plasmide agissant comme un élément compétitif multiple utilisable dans une amplification par PCR, contenant en séquence, séparés les uns des autres, deux éléments compétitifs ou plus homologues à ceux de la cible cellulaire, chaque élément compétitif contenant les séquences de reconnaissance d'une ou de deux paires d'amorces.

2. Plasmide selon la revendication 1, dans lequel chaque élément compétitif contient les séquences de reconnaissance d'une seule paire d'amorces.

3. Plasmide selon la revendication 2 contenant deux éléments compétitifs.

4. Plasmide selon la revendication 3, dans lequel un élément compétitif contient la paire d'amorces suivante : et le second élément compétitif contient la paire d'amorces suivante :

5. Plasmide selon la revendication 1, dans lequel chaque élément compétitif contient les séquences de reconnaissance de deux paires d'amorces.

6. Plasmide selon la revendication 5 contenant deux éléments compétitifs.

7. Plasmide selon la revendication 6, dans lequel un élément compétitif contient les deux paires d'amorces suivantes : (amorces externes)
et (amorces internes)
et le second élément compétitif contient les deux paires d'amorces suivantes : (amorces externes)
et (amorces externes)

8. Utilisation d'un plasmide selon la revendication 1 pour la détermination et la quantification simultanées de l'expression d'un ou de plusieurs gènes par des techniques de PCR compétitive.

9. Utilisation d'un plasmide selon la revendication 4 pour la détermination et la quantification simultanées de l'expression de type 1 et des récepteurs de type 2 de la somatostatine.

10. Utilisation du plasmide selon la revendication 7 pour la détermination et la quantification simultanées de l'expression des antigènes prostatiques PSA et PSM.

11. Procédé de détermination et de quantification simultanées de l'expression de deux gènes ou plus dans des techniques de PCR compétitives utilisant un plasmide selon la revendication 1.
